# EUROPEAN PATENT SPECIFICATION

(11) **EP 3 563 878 B1**
(45) Date of publication and mention of the grant of the patent: **16.11.2022**
(21) Application number: 17886250.4
(22) Date of filing: 14.12.2017
(51) Int. Cl.: H05H 1/24, H05H 1/46, A61L 9/22, A01K 1/00, B23K 9/09, H02M 1/00

(54) **AGRICULTURE AND STOCKBREEDING PLASMA GENERATION DEVICE USING RESONANT POWER DRIVER**
PLASMAERZEUGUNGSVORRICHTUNG FÜR LANDWIRTSCHAFT UND VIEHZUCHT UNTER VERWENDUNG EINES RESONANTEN LEISTUNGSTREIBERS
DISPOSITIF DE PRODUCTION DE PLASMA D'ÉLEVAGE ET D'AGRICULTURE UTILISANT UN DISPOSITIF RÉSONANT D'ALIMENTATION ÉLECTRIQUE

(30) Priority: 29.12.2016 KR 20160182186
(43) Date of publication of application: 06.11.2019
(73) Proprietor: Samdo Environmental Co., Ltd., Gwangju 62337 (KR)
(72) Inventor: JUNG, Woo Nam, Gwangju 61059 (KR)
(74) Representative: Mammel und Maser Patentanwälte PartG mbB
(86) International application number: PCT/KR2017/014708
(87) International publication number: WO 2018/124556

(56) References cited:
- EP-A1- 1 809 082
- EP-A2- 0 814 176
- WO-A1-2007/035216
- JP-A- 2010 240 630
- KR-A- 20110 067 615
- KR-A- 20160 047 574
- KR-B1- 100 956 844
- KR-B1- 101 317 355
- US-A1- 2002 153 241

## Description

### Technical Field

The present invention relates to an agriculture and stockbreeding plasma generation device using a resonant power driver, which is capable of more effectively removing various kinds of dust, ammonium nitrate (NH₄NO₃), and the like that are generated in livestock breeding facilities such as housing, livestock manure, etc.

### Background Art

Generally, in the agricultural and stockbreeding industry, odor, water pollution, and soil pollution, etc. caused by livestock manure are becoming a complex problem.

As a conventional method for removing odors, a method of regularly cleaning inside of the livestock housing is used. However, this method does not directly and efficiently control the odors from decomposition gas such as ammonia (NH₃), hydrogen sulfide (H₂S), and the like which are continuously generated from livestock and livestock manure, etc. in the livestock housing. Particularly, in Korea, there is a problem that the amount of harmful gas and odors generated due to long-term storage, transportation, or distributing of manure is increased due to the proximity of livestock housing to residential space. Odors caused by volatile fatty acid (VFA), ammonia, volatile amine, phenol, sulfur compounds, and so on, which cause environmental pollution inside the livestock housing and causes disease in livestock, is a serious problem.

Recently, various odor reduction technologies and facilities have been developed and provided to farmers. However, the ability to treat odors various among farmers using similar facilities, and it is not easy or practical to develop a complete prevention or remarkable abatement technology due to the characteristics of livestock odors. Furthermore, unlike a factory, all livestock housing facilities operate in form exposed to the outside, thus methods reducing odors are limited, and the cost of the odor prevention facility is considerably high. Therefore, it is difficult for farmers to install preventive facilities on their own, and the supply of facilities to farmhouses is also limited. In the case of single use of such a conventional odors treatment method, it is not efficient. Therefore, it is installed and processed in a complex manner. However, there are problems that the occupation area is wide, and the secondary pollution problem caused by chemical treatment and maintenance and management costs are excessive.

A conventional ozone generating apparatus collects and removes dust and other foreign substances contained in the ambient air by using negative ions. However, the conventional ozone generating device has a problem of generating ammonium nitrate in a solid state by binding with ammonia generated in the livestock house. Particularly, since ammonium nitrate is produced, there is a problem that the efficiency of generating ozone in the ozone generating apparatus is lowered.

KR 100 956 844 B1 discloses an apparatus and a method for processing bad smell using an adsorbent and plasma.

A method and an apparatus for producing diamond film by chemical vapor deposition is known from EP 0 814 176 A2.

It is known from EP 1 809 082 A1 to enhance the gas-excitation efficiency by replacing the protected electrode in conventional gas-exciting apparatus with an electrode containing an insulating coating layer.

Furthermore, WO 2007/035216 A1 discloses a resonant power supply for ozone generation, wherein a first AC voltage from a power source to a resonant circuit is provided and the resonant circuit provides a second AC voltage to the ozone generating unit, wherein the second AC voltage is greater than the first AC voltage.

A further example of a dielectric barrier discharge device for the purification of liquids and gases is disclosed in US 2002/153241 A1.

The techniques underlying the present invention are disclosed in the following documents.
Korean Patent Application Publication No. 10-1997-0065447 (1997.10.13)
Korean Utility Model Registration No. 20-0165592 (2000.02.15)
Korean Utility Model Registration No. 20-0319211 (2003.07.10)
Korean Patent No. 10-0954087 (2010.04.14)
Korean Patent Application Publication No. 10-2012-0021328(2012.03.09)
Korean Patent No. 10-1323046 (2013.10.29)
Korean Patent No. 10-1363282 (2014.02.10)

### Disclosure

### Technical Problem

The object of the present invention is to provide a plasma generator for agriculture and stockbreeding which can more effectively remove dust, ammonium nitrate and the like by maximizing the generation of ozone using a resonant power driver. Another object of the present invention is to provide a plasma generator for agriculture and stockbreeding using a resonant power driver, which is capable of removing odors, viruses, and the like generated by livestock manure through a plasma reaction.

### Technical Solution

The present invention provides an agriculture and stockbreeding plasma generation device according to claim 1.

In an example, the AC power source 210 may be configured to supply AC 220V (commercial power) as is or to drop it to AC 20V. The dropped AC 20V may be supplied to the second power supply circuit 220 or to the first power supply circuit 240 and the second power supply circuit 220.

In another example, the first power supply circuit 240 may be configured to convert AC 220V or AC 20V from the AC power source 210 to DC 15V and provide the DC voltage to the resonant circuit 250. The second power supply circuit 220 may be configured to convert AC 20V from the AC power source 210 to DC 15V and provide the DC voltage to the drive circuit 230.

In addition, as an example, the drive circuit 230 may be configured to convert DC 15V to AC 23,000V and provide it to the plasma generator 100. The control circuit 260 connected to the resonant circuit 250 and the drive circuit 230 controls the operation of the resonant circuit 250 when the plasma generator 100 is overloaded and the operation of the drive circuit 230.

### Advantageous Effects

The present invention can more effectively remove dust and ammonium nitrate, and can directly remove odors, viruses, and the like generated by livestock manure.

### Description of Drawings

FIG. 1 is a front view showing a plasma generator 100 of a plasma generation device for agriculture and stockbreeding according to the present invention.
FIG. 2 is a conceptual diagram showing the configuration of the resonant power driver 200 for driving the plasma generator 100 of the plasma generation device for agricultural and stockbreeding according to the present invention.

### Best Mode

Hereinafter, a preferred embodiment of the present invention will be described with reference to the accompanying drawings.

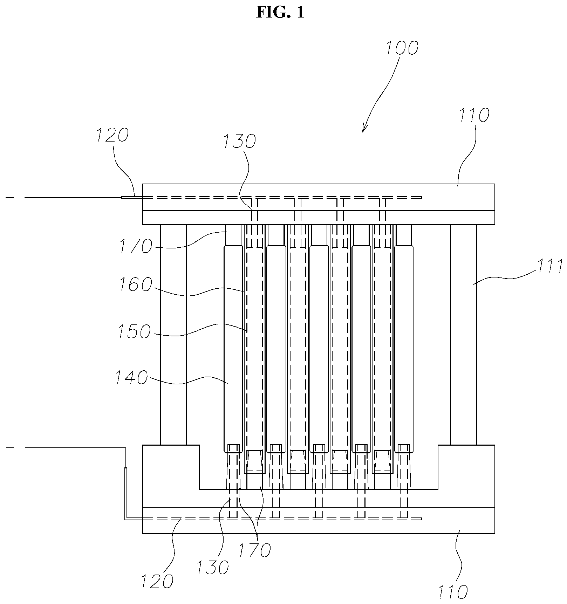

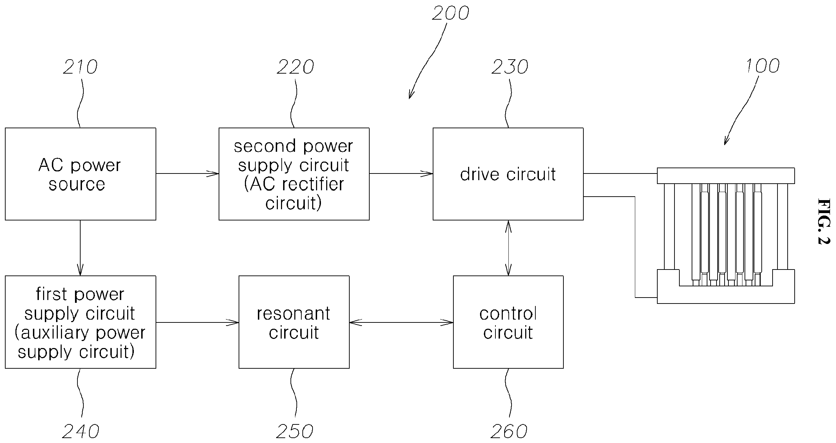

FIGS. 1 and 2 show a plasma generation device for agricultural and stockbreeding according to the present invention. As shown in FIGS. 1 and 2, the plasma generation device for agriculture and stockbreeding according to the present invention includes: a resonant power driver 200; and a plasma generator 100 for generating plasma by operation of the resonant power driver 200 to sterilize air generated from the housing, livestock manure, etc. and to remove odors.

The plasma generator 100 includes a pair of bodies 110 provided at upper and lower portions so as to be apart at regular intervals in a vertical direction; a power supply port 120 connected to the drive circuit 230 of the resonant power driver 200 and provided in each body 110 along the longitudinal direction (i.e., the horizontal direction) of the body 110; two or more electric conductors 140 and 150 connected to the power supply ports 120 and vertically installed between the pair of bodies 110 and arranged so as to be spaced apart from each other along the longitudinal direction of the body 110; and a ceramic tube 160 having the electric conductor 150 inserted therein by being fitted over the outer diameter (outer periphery) of the electrical conductor 150. The plasma generator 100 generates ozone by using a plasma discharge method and mixes the ozone with air to perform functions such as sterilization and odor removal.

The power supply ports 120 are connected to the drive circuit 230 and supply electric power to the electric conductors 140 and 150.

The ceramic tube 160, when power is supplied, generates ozone radicals by generating plasma between the electrical conductor 140 and the ceramic tube 160 and sterilizes or removes odors by mixing the ozone radical with air. The thickness of the ceramic tube 160 may be varied depending on the operating voltage and the spacing between the electrical conductor 140 and the ceramic tube 160.

An insulator 170 is provided between each of the bodies 110 and the electrical conductors 140 and 150. Using the insulator 170, the bodies 110 and the electrical conductors 140 and 150 are coupled together in an insulated state. A connection port 130 is provided between each of the power supply ports 120 and the electrical conductors 140 and 150 to supply power easily.

Ammonium nitrate can be removed by washing by spraying wash water toward the surface of the electrical conductor 140 and the ceramic tube 160. For this purpose, a spraying nozzle is provided so as to face the surface of the electrical conductor 140 and the ceramic tube 160. And a pump connected to the spraying nozzle to supply wash water may be included. According to this, by supplying the wash water to the spraying nozzle by the operation of the pump, foreign matter, ammonium nitrate, etc. generated on the surfaces of the electric conductor 140 and the ceramic tube 160 can be removed.

The resonant power driver 200 adjusts the oscillation frequency and the resonance state so that the plasma generator 100 can generate ozone in an optimal state.

Referring to FIG. 2, the resonant power driver 200 includes an AC power source 210 for supplying AC power; a second power supply circuit 220 connected to the AC power source 210 and rectifying the AC power and converting the AC power to DC power; a drive circuit 230 connected to the second power supply circuit 220 for generating an output by amplifying the current voltage; a first power supply circuit 240 for supplying power to the resonant circuit 250 and the control circuit 260; a resonant circuit 250 for generating a resonant waveform; and a control circuit 260 connected to the resonant circuit 250 and controlling the resonant circuit 250 when an excessive output of the plasma generator 100 is generated.

AC 180V to AC 260V is used for smooth operation of the plasma generator 100. The AC power source 210 may be configured to make AC 180V to AC 260V (preferably AC 220V) as a good quality power source and also to drop to, for example, AC 20V. Accordingly, the AC power source 210 may include a filter that removes noise from the AC power source, thereby preventing damage to each component or the like and prevent high frequency noise from adversely affecting each component.

For example, the second power supply circuit 220 may be configured to convert AC 20V to DC 15V. The second power supply circuit 220 performing the function of the AC rectification circuit may include a condenser for converting the AC power to the DC power. For the operation of the plasma generator 100, a half bridge and a rectification capacitor may be used.

The drive circuit 230 transmits the resonant drive power to the plasma generator 100 by boosting the resonance frequency. The drive circuit 230 serves as a resonant element.

The first power supply circuit 240 connected to both the resonant circuit 250 and the control circuit 260 performs a role of power supply.

The resonant circuit 250 can generate a stable resonant frequency and waveform by using an IC-type resonant circuit. To set the optimum resonant frequency, by using multi-turn volume, it can be easily adjusted without jittering when making fine adjustments.

It is provided that the resonance can be blocked and recovered by a signal passed from the control circuit 260. The control circuit 260 generates a signal so that the resonant circuit 250 can be cut off when an excessive output is generated in the plasma generator 100. When an abnormality occurs in the control circuit 260 or no output is outputted due to a failure in connection with the control circuit 260, an alarm signal is generated to the outside so that a manager can easily recognize the alarm status. At this time, an operation lamp may be installed to indicate whether the operation is normal or not.

## Claims

1. An agriculture and stockbreeding plasma generation device using a resonant power driver, the device comprising:
a resonant power driver (200); and
a plasma generator (100) for generating plasma by operation of the resonant power driver (200) to sterilize air and remove odors, wherein
the resonant power driver (200) includes:
an AC power source (210) for supplying AC power;
a first power supply circuit (240) connected to the AC power source (210);
a resonant circuit (250) for receiving power from the first power supply circuit (240) and generating a resonant waveform;
a drive circuit (230) for amplifying the output of the resonant circuit (250) and supplying the amplified output to the plasma generator (100);
a second power supply circuit (220) for converting AC power from the AC power source (210) to DC power and supplying driving power to the drive circuit (230); and
a control circuit (260) for controlling at least one or more of the resonant circuit (250) and the drive circuit (230) according to an operation state of the plasma generator (100), and
the plasma generator (100) includes:
a pair of bodies (110) arranged on top and bottom of the device and being spaced apart from each other by a gap in a vertical direction;
a power supply port (120) provided along a longitudinal direction of each of the bodies (110) and electrically connected to the drive circuit (230);
a plurality of first and second electrical conductors (140 and 150) connected to the power supply port (120) and vertically arranged along the longitudinal directions of the bodies (110) while being spaced apart from each other by gaps;
a ceramic tube (160) fitted over an outer diameter of the second electrical conductors (150) and, when power is supplied, configured to generate ozone radicals by generation of plasma between the first electrical conductors (140) and the ceramic tube (160);
a spraying nozzle provided to face the surface of the first electrical conductor (140) and the ceramic tube (160) and configured to spray washing water toward the surface of the first electrical conductor (140) and the ceramic tube (160) ;
an insulator (170) provided between the bodies (110) and the plurality of first and second electrical conductors (140 and 150); and
a connector (130) provided between the power supply port (120) and the plurality of first and second electrical conductors (140 and 150)

## Patentansprüche

1. Plasmaerzeugungsvorrichtung für Landwirtschaft und Viehzucht unter Verwendung eines resonanten Leistungstreibers, wobei die Vorrichtung umfasst:
einen resonanten Leistungstreiber (200); und
einen Plasmagenerator (100), um durch den Betrieb des resonanten Leistungstreibers (200) Plasma zur Luftsterilisierung und zur Geruchsbeseitigung zu erzeugen, wobei
der resonante Leistungstreiber (200) enthält:
eine AC-Stromquelle (210) zur Versorgung mit AC-Strom;
eine erste Stromversorgungsschaltung (240), die mit der AC-Stromquelle (210) verbunden ist;
eine resonante Schaltung (250) zur Aufnahme von Strom von der ersten Stromversorgungsschaltung (240) und zum Erzeugen einer resonanten Wellenform;
eine Antriebsschaltung (230) zum Verstärken der Leistungsabgabe der resonanten Schaltung (250) und zum Zuführen der verstärkten Leistungsabgabe an den Plasmagenerator (100);
eine zweite Stromversorgungsschaltung (220) zum Umwandeln von AC-Strom aus der AC-Stromquelle (210) in DC-Strom und zum Zuführen von Antriebsleistung an die Antriebsschaltung (230); und
eine Steuerungsschaltung (260) zum Ansteuern zumindest der resonanten Schaltung (250) und/oder der Antriebsschaltung (230) in Übereinstimmung mit dem Betriebszustand des Plasmagenerators (100), und
der Plasmagenerator (100) enthält:
ein Paar von Körpern (110), die oben und unten an der Vorrichtung angebracht sind und in einer vertikalen Richtung durch einen Spalt voneinander beabstandet sind;
einen Stromversorgungsanschluss (120), der entlang einer Längsrichtung eines jeden der Körper (110) vorgesehen ist und der elektrisch mit der Antriebsschaltung (230) verbunden ist;
eine Mehrzahl erster und zweiter elektrischer Leiter (140 und 150), die mit dem Stromversorgungsanschluss (120) verbunden sind und derart entlang der Längsrichtungen der Körper (110) angeordnet sind, dass sie durch Spalte vertikal voneinander beabstandet sind;
ein Keramikrohr (160), das auf einen Außendurchmesser der zweiten elektrischen Leiter (150) aufgesetzt ist und dafür ausgelegt ist, im bestromten Zustand Ozonradikale zu erzeugen, indem zwischen den ersten elektrischen Leitern (140) und dem Keramikrohr (160) Plasma erzeugt wird;
eine Sprühdüse, welche derart vorgesehen ist, dass sie zu der Oberfläche des ersten elektrischen Leiters (140) und des Keramikrohrs (160) hin ausgerichtet ist, und welche dafür ausgelegt ist, Waschwasser auf die Oberfläche des ersten elektrischen Leiters (140) und des Keramikrohrs (160) zu sprühen;
einen Isolator (170), welcher zwischen den Körpern (110) und der Mehrzahl von ersten und zweiten elektrischen Leitern (140 und 150) vorgesehen ist; und
ein Verbindungselement (130), welches zwischen dem Stromversorgungsanschluss (120) und der Mehrzahl von ersten und zweiten elektrischen Leitern (140 und 150) vorgesehen ist.

## Revendications

1. Dispositif de génération de plasma pour l'agriculture et l'élevage utilisant un pilote de puissance résonant, ledit dispositif comprenant :
un pilote de puissance résonnant (200) et
un générateur de plasma (100) afin de générer, grâce au fonctionnement du pilote de puissance résonant (200), du plasma pour la stérilisation de l'air et l'élimination des odeurs,
le pilote de puissance résonant (200) comportant :
une source de courant AC (210) pour l'alimentation en courant AC ;
un premier circuit d'alimentation électrique (240) qui est relié à la source de courant AC (210) ;
un circuit résonant (250) destiné à recevoir du courant provenant du premier circuit d'alimentation électrique (240) et à générer une forme d'onde résonante ;
un circuit d'entraînement (230) destiné à amplifier la puissance de sortie du circuit résonant (250) et à fournir la puissance de sortie amplifiée au générateur de plasma (100) ;
un deuxième circuit d'alimentation électrique (220) destiné à transformer du courant AC provenant de la source de courant AC (210) en courant DC et à fournir de la puissance d'entraînement au circuit d'entraînement (230) ; et
un circuit de commande (260) destiné à commander au moins le circuit résonant (250) et/ou le circuit d'entraînement (230) conformément à un état de fonctionnement du générateur de plasma (100), et
le générateur de plasma (100) comportant :
une paire de corps (110) qui sont disposés en haut et en bas du dispositif et qui sont espacés l'un de l'autre en sens vertical par une fente ;
une borne d'alimentation électrique (120) qui est prévue le long d'un sens longitudinal de chacun des corps (110) et qui est reliée électriquement au circuit d'entraînement (230) ;
une pluralité de premiers et de deuxièmes conducteurs électriques (140 et 150) qui sont reliés à la borne d'alimentation électrique (120) et qui sont disposés le long des sens longitudinaux des corps (110) de manière à être espacés verticalement les uns des autres par des fentes ;
un tube de céramique (160) qui est placé sur un diamètre extérieur des deuxièmes conducteurs électriques (150) et qui est conçu, lorsqu'il est alimenté en courant, pour produire des radicaux d'ozone en générant du plasma entre les premiers conducteurs électriques (140) et le tube de céramique (160) ;
une buse de pulvérisation qui est prévue de manière à être orientée vers la surface du premier conducteur électrique (140) et du tube de céramique (160), et qui est conçue pour pulvériser de l'eau de lavage sur la surface du premier conducteur électrique (140) et du tube de céramique (160) ;
un isolateur (170) qui est prévu entre les corps (110) et la pluralité de premiers et de deuxièmes conducteurs électriques (140 et 150) ; et
un connecteur (130) qui est prévu entre la borne d'alimentation électrique (120) et la pluralité de premiers et de deuxièmes conducteurs électriques (140 et 150).
